# EUROPÄISCHE PATENTSCHRIFT

(11) **EP 2 361 040 B1**
(45) Veröffentlichungstag und Bekanntmachung des Hinweises auf die Patenterteilung: **06.01.2021**
(21) Anmeldenummer: 09763936.3
(22) Anmeldetag: 27.11.2009
(51) Int. Cl.: A61B 17/00, A61B 17/29

(54) **INSTRUMENT FÜR DIE LAPAROSKOPISCHE CHIRURGIE**
INSTRUMENT FOR LAPAROSCOPIC SURGERY
INSTRUMENT POUR CHIRURGIE LAPAROSCOPIQUE

(30) Priorität: 27.11.2008 DE 202008015763 U
(43) Veröffentlichungstag der Anmeldung: 31.08.2011
(73) Patentinhaber: Q Medical International AG, 8259 Kaltenbach (CH)
(72) Erfinder: TIMMERMAN, Andre, NL-1336 SG Almere (NL)
(74) Vertreter: dompatent von Kreisler Selting Werner - Partnerschaft von Patent- und Rechtsanwälten mbB
(86) Internationale Anmeldenummer: PCT/EP2009/065996
(87) Internationale Veröffentlichungsnummer: WO 2010/060992

(56) Entgegenhaltungen:
- EP-A1- 1 584 293
- EP-A2- 2 656 777
- WO-A1-98/42260
- US-A- 5 618 294
- US-A- 5 885 288
- US-A- 5 919 199

## Beschreibung

Die Erfindung betrifft ein Instrument für die laparoskopische Chirurgie nach dem Oberbegriff des Anspruchs 1.

Die Laparoskopie, auch Bauchspiegelung genannt, bezeichnet eine Methode, bei der die Bauchhöhle und die darin liegenden Organe mit speziellen Endoskopen durch eine kleine, vom Chirurgen geschaffene Öffnung in der Bauchdecke sichtbar gemacht werden. Über einen Hautschnitt wird ein sogenannter Trokar in die Bauchdecke eingebracht, durch den mit Hilfe eines Laparoskops, das an eine Videokamera und an eine Lichtquelle angeschlossen ist, der Bauchraum eingesehen werden kann.

Bei einem operativen Eingriff werden über weitere Hautschnitte zusätzliche Torkare eingebracht, durch die Instrumente zur Laparoskopie in dem Bauchraum eingeführt werden können, mit deren Hilfe die Operation durchgeführt werden kann.

Es sind die Instrumente für die laparoskopische Chirurgie bekannt, die beispielsweise in geradliniger Form durch den Trokar in den Bauchraum eingeführt werden, und die innerhalb des Bauchraums bestimmte Funktionen ausüben können.

Hierzu weisen die Instrumente einen Bedienungshandgriff auf, der es erlaubt, von außen die Funktionen auszuführen.

Mit dem Bedienungshandgriff ist zunächst ein geradliniger erster Schaftabschnitt verbunden. Bei einem dieser Instrumente ist es vorgesehen, dass sich an den ersten Schaftabschnitt ein weiterer biegsamer Schaftabschnitt anschließt, der mit Hilfe eines Zugmittels von einer geradlinigen Streckposition in eine gebogene Position in einer vorbestimmten Krümmungsebene überführbar ist. Am distalen Ende des zweiten Schaftabschnitts befindet sich ein Endteil, das ggf. mit Zusatzfunktionen ausgestattet sein kann.

Die US 5618294 offenbart ein Instrument mit einem Bedienungshandgriff, der mit einem geradlinigen ersten Schaftabschnitt verbunden ist. Es ist vorgesehen dass sich an den ersten Schaftabschnitt ein weiterer biegsamer Schaftabschnitt anschließt, der mit Hilfe eines Zugmittels von einer geradlinigen Streckposition in eine gebogene Position in einer vorbestimmten Krümmungsebene überführbar ist. Am distalen Ende des zweiten Schaftabschnitts befindet sich ein Endteil, das ggf. mit Zusatzfunktionen ausgestattet sein kann.

Die EP 1 584 293 A1 offenbart Instrument mit einem Betätigungselement (5), das in der Nähe eines Griffs (1) angeordnet ist, und mit einem Zugelement (7) zusammenwirkt. Eine Blattfeder erstreckt sich in einem nicht gespannten Zustand in einem geraden Verlauf in Längsrichtung über einen krümmbaren Abschnitt (4). Führungshalterungen sind in Längsrichtung des gekrümmten Abschnitts voneinander beabstandet angeordnet, um das Anheben des Zugelements von der Blattfeder zu begrenzen.

Die WO98/42260 offenbart eine laparoskopische Applikatorvorrichtung (10) zum selektiven gerichteten Aufbringen einer oder mehrerer Flüssigkeiten auf eine Operationsstelle. Die Vorrichtung (10) umfasst einen Griff (12) mit Flüssigkeitseinlässen (16, 18, 20) an einem Ende, diskreten Kanälen innerhalb eines oder mehrerer Rohre (21), die mit den Einlässen (16, 18, 20) verbunden sind und sich durch den Griff zu einer Düse (22) erstrecken (12) und. Es existiert ein starrer Abschnitt unmittelbar neben dem Griff (12) und einen flexibler Abschnitt nahe der Düsenspitze.

Das Endteil ist mit zwei Zugseilen verbunden, die mit Hilfe eines Drehgriffs am Bedienungshandgriff gespannt werden können, indem die Länge der Zugseile verkürzt wird, so dass sich der biegsame Schaftabschnitt in einer vorgegebenen Krümmungsebene biegen kann. Der biegbare zweite Schaftabschnitt besteht dabei aus Einzelsegmenten, die Anschlagflächen aufweisen und damit die Biegung des zweiten Schaftabschnittes begrenzen. Soll das Instrument aus der gebogenen Endposition wieder in die Strecklage gebracht werden, muss wiederum der Drehgriff an dem Bedienungshandgriff betätigt werden, wodurch die Länge des Zugseils vergrößert wird und der zweite Schaftabschnitt in seine Strecklage verbracht werden kann. Nachteilig ist dabei, dass eine beidhändige Betätigung des Instrumentes erforderlich ist. Desweiteren ist nachteilig, dass sich die Festigkeit des zweiten Schaftabschnitts seitlich der Krümmungsebene erst einstellt, wenn die einzelnen Segmente an ihren Anschlägen anliegen.

Der Erfindung liegt die Aufgabe zugrunde, ein Instrument der eingangs genannten Art derart weiterzubilden, dass eine Einhandbedienung möglich ist, und dass eine höhere Seitenstabilität auch im Zwischenpositionen des biegsamen Schaftabschnitts erreicht wird.

Zur Lösung dieser Aufgabe dienen die Merkmale des Anspruchs 1.

Die Erfindung sieht vor, dass sich zwischen dem ersten Schaftabschnitt und dem Endteil ein flexibles Verbindungsmittel erstreckt, und dass das flexible Verbindungsmittel eine Rückstellfunktion aufweist, mit der der biegsame zweite Schaftabschnitt selbsttätig in eine geradlinige oder vorgegebene Streckposition rückstellbar ist.

Die Einhandbedienung ermöglicht es, das Instrument einzuführen und dabei gleichzeitig im Bauchraum die Krümmung des zweiten Schaftabschnittes in der gewünschten Weise mit einer Hand auszuführen.

Aufgrund des flexiblen Verbindungsmittels, das eine Rückstellfunktion aufweist kann der Grad der Krümmung des biegbaren Schaftabschnittes ständig verändert werden und an die momentanen Anforderungen angepasst werden, ohne dass die zweite Hand für die Verstellung der Krümmung eingesetzt werden muss. Dadurch besteht die Möglichkeit, die zweite Hand für andere Tätigkeiten im Bauchraum mit einem zweiten Instrument zu nutzen. Aufgrund des flexiblen Verbindungsmittels, das zusätzlich zu dem Zugmittel vorgesehen ist, weist das Instrument in dem zweiten Schaftabschnitt auch eine höhere Seitenstabilität auf, d.h. in Richtung orthogonal zu der Krümmungsebene des zweiten Schaftabschnittes.

Das Verbindungsmittel kann an dem ersten Schaftabschnitt und dem Endteil starr oder lösbar befestigt sein. An dem distalen Ende des ersten Schaftabschnittes kann eine Kopplungseinrichtung vorgesehen sein, die es ermöglicht, den zweiten Schaftabschnitt auszuwechseln. Das Kopplungsteil verbindet dabei das Zugmittel mit einer Betätigungseinrichtung des Bedienungshandgriffs, mit dem das Zugmittel vor- und zurückbewegt werden kann. Die Möglichkeit, den biegbaren Schaftabschnitt von dem geradlinigen ersten Schaftabschnitt abzutrennen, erlaubt es, den zweiten Schaftabschnitt als Wegwerfteil zu konzipieren, so dass die Sterilisation des Instrumentes vereinfacht ist.

Gemäß der vorliegenden Erfindung ist der zweite Schaftabschnitt zwischen dem ersten Schaftabschnitt und dem Endteil aus mehreren gelenkig zueinander beweglichen Einzelsegmenten gebildet.

Dabei ist vorgesehen, dass die Einzelsegmente einen gegenseitigen Abstand aufweisen und auch in der gebogenen Endposition mit ihren einander gegenüberliegenden Flächen nicht aneinanderstoßen.

Das Verbindungsmittel ist vorzugsweise ein Federelement, das selbsttätig in die ursprünglich geradlinige Streckposition zurückkehrt, wenn das Zugmittel keine Spannung auf den zweiten Schaftabschnitt ausübt. Das Federelement kann z.B. aus einem elastischen Stahldraht bestehen.

Vorzugsweise besteht das Verbindungsmittel aus einem Metall mit Memory-Effekt, z.B. Nitinol. Alternativ kann das Verbindungsmittel ein Metall mit Memory-Effekt enthalten.

Ein derartiges Memory-Metall zeigt ein pseudoelastisches Verhalten, bei dem das Material beim Entlasten durch seine innere Spannung wieder in seine Ursprungsform zurückkehrt.

Das Verbindungsmittel kann aus mindestens zwei seitlich beabstandeten Elementen, z.B. Drähten bestehen.

Das Verbindungsmittel kann an der in gebogenem Zustand äußeren Seite der Einzelsegmente angeordnet sein.

Gemäß der vorliegenden Erfindung weisen die Einzelsegmente Fußteile auf, wobei das Verbindungsmittel durch das Fußteil des Einzelsegmentes hindurchgeführt ist.

Das Verbindungsmittel kann dabei an dem Endteil sowie an dem ersten Einzelsegment befestigt sein.

Das Zugmittel ist dabei auf der von dem Verbindungsmittel entfernten Seite der Einzelsegmente durch diese Einzelsegmente hindurchgeführt.

Der maximale Verschiebungsweg des Zugmittels kann durch ein Betätigungsmittel im Bedienungshandgriff begrenzt sein. Das Betätigungsmittel besteht beispielsweise aus einem Schwenkhebel an dem Bedienungshandgriff, der beim Halten des Handgriffs mit einem Finger bedient werden kann.

Der maximale Verschiebungsweg des Zugmittels, der den Grad der Krümmung des zweiten Schaftabschnitts bestimmt, kann variabel an dem Betätigungsmittel einstellbar sein.

Es werden daher keine Anschläge an den Einzelsegmenten benötigt. Vorzugsweise ist eine Arretiervorrichtung an dem Bedienungshandgriff vorgesehen, mit der in wählbaren Zugpositionen das Zugmittel arretiert werden kann. Dies bedeutet, dass der biegsame zweite Schaftabschnitt in jeder gewünschten Krümmungslage mit Hilfe der Arretiervorrichtung festgestellt werden kann, so dass der Chirurg alle Zwischenpositionen zwischen der Strecklage und der gekrümmten Endposition einstellen kann. Die Arretiervorrichtung ist ebenfalls an dem Bedienungshandgriff angeordnet.

Die Fußteile der Einzelsegmente liegen aneinander und bilden bei der Krümmung des zweiten Schaftabschnitts ein Gelenk. Dabei sind die Einzelsegmente bis auf das erste und letzte nicht an dem Verbindungsmittel befestigt und insofern beweglich auf dem Verbindungsmittel verschiebbar.

In einem nicht zu der Erfindung gehörendes Ausführungsbeispiel kann der zweite Schaftabschnitt anstelle von Einzelsegmenten aus einem flexiblen Vollmaterial oder aus einem flexiblen Schlauchmaterial gebildet sein. Dabei kann das Verbindungsmittel entweder außerhalb an dem Voll- oder Schlauchmaterial angeordnet sein, oder innerhalb des Vollmaterials bzw. des Schlauchmaterials, während das Zugmittel stets innerhalb des Voll- oder Schlauchmaterials geführt ist.

Beispielsweise kann das Voll- oder Schlauchmaterial aus Silikon bestehen. Im Falle der Ausführung des zweiten Schaftabschnitts aus einem flexiblen Vollmaterial oder aus einem flexiblem Schlauchmaterial kann das Verbindungsmittel aus einem dünnen, flexiblen Streifen bestehen, durch den die Krümmungsebene in Verbindung mit dem Zugmittel vorgegeben ist, wobei dessen Rückstellkraft so bemessen ist, dass auch das flexible Vollmaterial oder das flexible Schlauchmaterial in die Strecklage zurückgeführt werden kann, wenn das Zugmittel keine Kräfte mehr ausübt.

Im folgenden werden unter Bezugnahme auf die Zeichnungen Ausführungsbeispiele der Erfindung näher erläutert.

Es zeigen:
- Fig. 1: das laparoskopische Instrument,
- Fig. 2: einen Schnitt durch den Handgriff im Bereich der Betätigungseinrichtung,
- Fig. 3: ein erstes Ausführungsbeispiel des biegbaren Schaftabschnitts,
- Fig. 4: ein zweites nicht zu der Erfindung gehörendes Ausführungsbeispiel für einen biegbaren Schaftabschnitt aus elastischem Kunststoffmaterial,
- Fig. 5: ein Einzelsegment des biegbaren Schaftabschnitts in einer Seitenansicht, und
- Fig. 6: eine stirnseitige Ansicht des Einzelsegmentes gemäß Fig. 5.

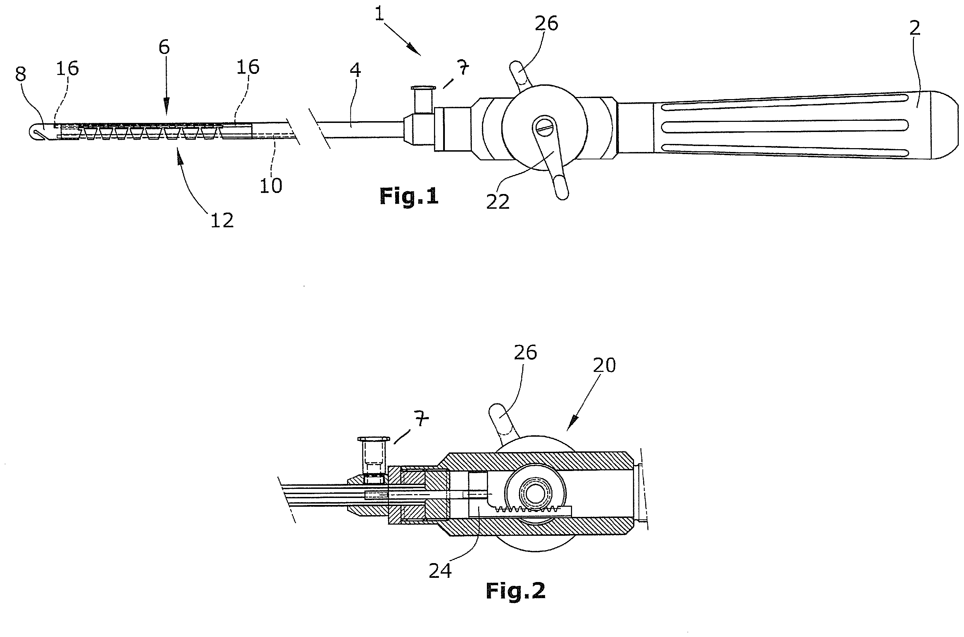

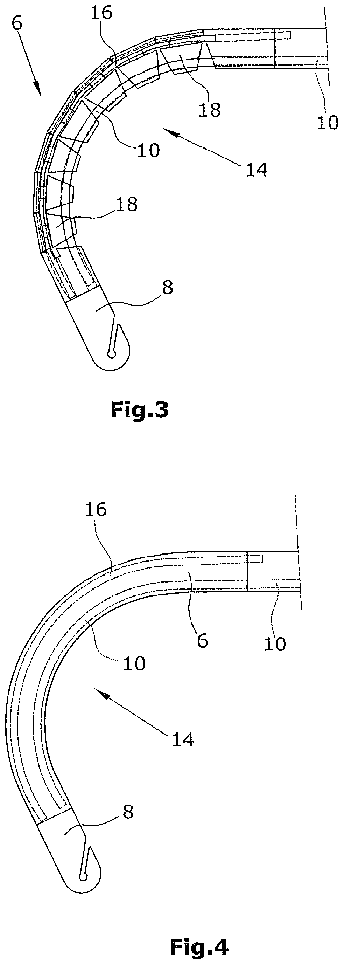

Das in Fig. 1 gezeigte laparoskopische Instrument 1 weist einen Bedienungshandgriff 2 auf, wobei der Bedienungshandgriff 2 mit einem geradlinigen ersten Schaftabschnitt 4 verbunden ist. In distaler Richtung schließt sich ein biegbarer zweiter Schaftabschnitt 6 am distalen Ende des ersten Schaftabschnittes 4 an, der ein an der Spitze des zweiten Schaftabschnittes 6 befindliches Endteil 8 aufweist. Ein Zugmittel 10 greift einerseits an dem Endteil 8 an und andererseits an einer Betätigungseinrichtung 20 mit einem Schwenkhebel 22 und einer Zahnstange 24 mit deren Hilfe das Zugmittel 10 von einer geradlinigen Streckposition 12, wie aus Fig. 1 ersichtlich, in eine gebogene Position 14 überführt werden kann, wie sie aus den Fign. 3 und 4 ersichtlich ist.

Das Zugmittel 10, z.B. ein Zugseil, ist dabei an der Zahnstange 24 angekoppelt. Mit dem Verschwenken des Schwenkhebels 22 kann demzufolge das Zugmittel 10 gespannt werden, wodurch sich der biegsame Schaftabschnitt 6 krümmt, wie aus den Fign. 3 und 4 ersichtlich.

Zwischen dem ersten Schaftabschnitt 4 und dem Endteil 8 erstreckt sich ein flexibles Verbindungsmittel 16, das an dem Endteil 8 und an dem ersten Schaftabschnitt 4 befestigt ist.

Das flexible Verbindungsmittel 16 weist eine Rückstellfunktion auf, mit der der biegsame zweite Schaftabschnitt 6 selbsttätig in die geradlinige Streckposition 12 oder eine vorgegebene Streckposition rückstellbar ist.

An dem Ende des ersten Schaftabschnitts 4, das dem Bedienungshandgriff 2 zugewandt ist, ist ein Anschlußstutzen 7 für eine Spüllösung angeordnet. Der Anschlußstutzen 7 weist eine durchgehende Bohrung auf. Die Bohrung endet in dem hohlen ersten Schaftabschnitt 4. Der erste Schaftabschnitt 4 und ein Teil des zweiten Schaftabschnitts 6 können über den Anschlußstutzen 7 mit einer Spüllösung gespült werden. Dabei tritt die Spüllösung über den Anschlußstutzen 7 in den ersten Schaftabschnitt 4 ein, durchspült den ersten Schaftabschnitt 4 und teilweise den in dem zweiten Schaftabschnitts 6 angeordneten Kanal 34, um dann bei dem Übergang zum ersten Einzelsegment 18 auszutreten.

Bei dem Ausführungsbeispiel der Fig. 3 ist der zweite Schaftabschnitt aus mehreren gelenkig zueinander beweglichen Einzelsegmenten 18 gebildet.

Die Einzelsegmente 18 weisen einen gegenseitigen Abstand auf.

Auch in der gebogenen Endposition 14 weisen die Einzelsegmente 18 einen gegenseitigen Abstand auf.

Figur 4 zeigt ein zweites nicht zu der Erfindung gehörendes Ausführungsbeispiel, bei dem der zweite Schaftabschnitt 6 aus einem elastischen Material, zum Beispiel Silikon gebildet ist, wobei das Material aus Vollmaterial bestehen kann oder aus einem Schlauchmaterial. Im Falle eines Vollmaterials kann das Verbindungsmittel 16 vollständig von dem Silikonmaterial umschlossen sein, während das Zugmittel 10 in einem Kanal in dem Vollmaterial geführt ist.

Im Falle eines Schlauchmaterials sollte das Verbindungsmittel 16 an mindestens zwei Stellen an der Innenseite oder Außenseite des Schlauchsmaterials befestigt sein, während das Zugmittel 10 im Inneren des Schlauchmaterials frei beweglich hindurchgeführt sein kann.

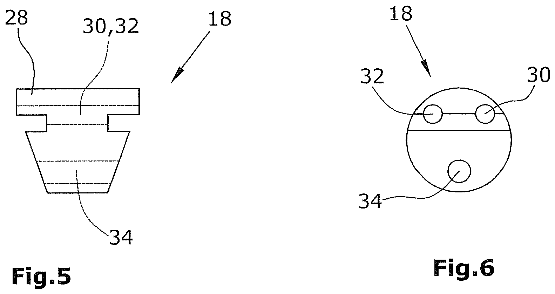

Die Figuren 5 und 6 zeigen ein Einzelsegment 18. Die in Figur 5 ersichtliche Seitenansicht zeigt, dass das Einzelsegment 18 eine im Wesentlichen trapezförmige Form aufweist, die sich in der Krümmungsebene des zweites Schaftabschnitts 6 nach innen verjüngt. Das Einzelsegment 18 weist ein Fußteil 28 mit zwei Kanälen 30 und 32 auf, durch die jeweils ein Verbindungsmittel 16 verläuft. In dem trapezförmigen Teil des Einzelsegments 18 ist ein weiterer Kanal 34 vorgesehen, durch den das Zugmittel 10 hindurchgeführt ist.

Das Verbindungsmittel 16 besteht vorzugsweise aus einem Metall mit Memory-Effekt oder weist ein Metall mit Memory-Effekt auf. Ein derartiges Metall ist beispielsweise Nitinol.

An dem Handgriff 2 ist, wie am besten aus den Figuren 1 und 2 ersichtlich, des Weiteren eine Arretiervorrichtung 26 vorgesehen, die das Zugmittel 10 in beliebig wählbaren Zugpositionen feststellt. Das Instrument 1 ermöglicht somit die Einhandbedienung, wobei mit dem Schwenkhebel 22 das Zugmittel 10 betätigt wird und mit dem weiteren Schwenkhebel der Arettiervorrichtung 26, der Schwenkhebel 22 in jeder gewüschten Stellung arretiert werden kann. Dabei begrenzt die Länge der Zahnstange 24 die maximale Krümmung des zweiten Schaftabschnitts 6.

Gemäß einer bevorzugten Ausführungsform ist der zweite biegsame Schaftabschnitt 6 von dem ersten Schaftabschnitt 4 abtrennbar, sodass der zweite Schaftabschnitt auswechselbar an dem ersten Schaftabschnitt 4 befestigt ist. Eine in den Zeichnungen nicht gezeigte Kupplungseinrichtung verbindet dabei das Zugmittel 10 des zweiten Schaftabschnitts 6 mit der Betätigungseinrichtung.

Da der zweite Schaftabschnitt 4 als Wegwerfteil konzipiert werden kann, ist das Instrument insgesamt einfacher nach jeder Benutzung zu sterilisieren.

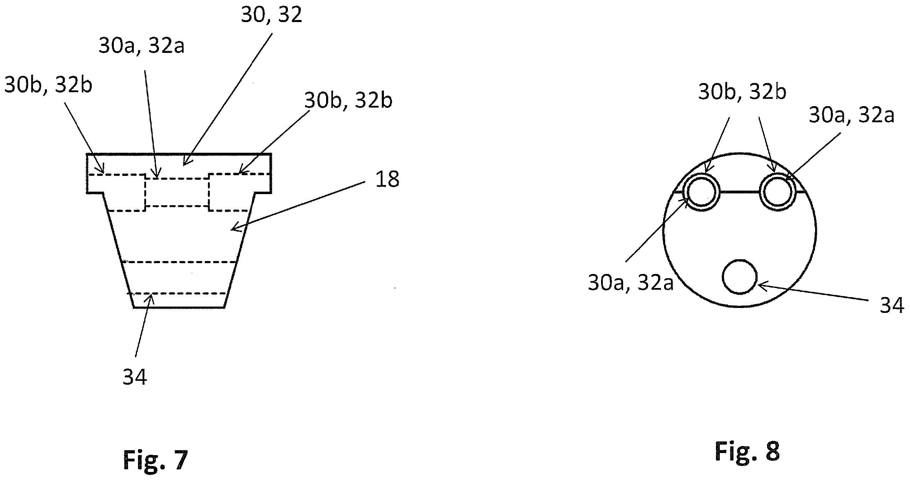

Die Figuren 7 und 8 zeigen eine weitere Ausführungsform der Einzelsegmente 18. Die in den Figuren 7 und 8 dargestellten Einzelsegmente 18 sind den Einzelsegmenten 18 aus den Figuren 5 und 6 sehr ähnlich. Die Einzelsegmente 18 weisen ebenfalls zwei Kanäle 30, 32 für jeweils ein flexibles Verbindungselement 16 und ein Kanal 34 für das Zugmittel 10 auf.

Die Kanäle 30, 32 für die flexiblen Verbindungselemente 16 weisen jedoch jeweils einen ersten Teilabschnitt 30a, 32a und jeweils zwei zweite Teilabschnitte 30b, 32b auf, wobei der jeweils erste Teilabschnitt 30a, 32a einen kleineren Durchmesser aufweist als die jeweils zwei zweiten Teilabschnitte 30b, 32b. Der jeweils erste Teilabschnitt 30a, 32a ist zwischen den jeweils zwei zweiten Teilabschnitten 30b, 32b angeordnet.

## Patentansprüche

1. Instrument (1) für die laparoskopische Chirurgie, mit
- einem Bedienungshandgriff (2),
- einem mit dem Bedienungshandgriff (2) verbundenen geradlinigen ersten Schaftabschnitt (4),
- einem biegbaren zweiten Schaftabschnitt (6) am distalen Ende des ersten Schaftabschnittes (4), der ein an der distalen Spitze des zweiten Schaftabschnitts (6) befindliches Endteil (8) aufweist, und
- einem Zugmittel, wobei
- der zweite Schaftabschnitt (6) mit Hilfe des an dem Endteil (8) angreifenden Zugmittels (10) von einer geradlinigen Streckposition (12) in eine gebogene Position (14) in einer vorgegebenen Krümmungsebene überführbar ist,
wobei der zweite Schaftabschnitt (6) zwischen dem ersten Schaftabschnitt (4) und dem Endteil (8) aus mehreren zusammengesetzten gelenkig zueinander beweglichen Einzelsegmenten (18) gebildet ist, wobei an jedem Einzelsegment (18) ein Fußteil (28) angeordnet ist, wobei die Fußteile (28) der Einzelsegmente (18) ein Gelenk bildend aneinanderliegen, und
wobei das Instrument derart ausgebildet ist, dass das Instrument in dem zweiten Schaftabschnitt (6) in Richtung orthogonal zu der Krümmungsebene des zweiten Schaftabschnitts (6) eine höhere Seitenstabilität als in Richtung der Krümmungsebene aufweist
**dadurch gekennzeichnet,dass**
sich zwischen dem ersten Schaftabschnitt (4) und dem Endteil (8) ein flexibles Verbindungsmittel (16) erstreckt, und
das flexible Verbindungsmittel (16) eine Rückstellfunktion aufweist, mit der der biegsame zweite Schaftabschnitt (6) selbsttätig in eine geradlinige oder vorgegebene Streckposition rückstellbar ist,
wobei das Verbindungsmittel (16) durch die Fußteile (28) der Einzelsegmente (18) hindurchgeführt ist.

2. Instrument nach Anspruch 1, **dadurch gekennzeichnet, dass** das Verbindungsmittel (16) an dem ersten Schaftabschnitt (4) und dem Endteil (8) starr befestigt ist.

3. Instrument nach Anspruch 1 oder 2, **dadurch gekennzeichnet, dass** die Einzelsegmente (18) einen gegenseitigen Abstand aufweisen.

4. Instrument nach einem der Ansprüche 1 bis 3, **dadurch gekennzeichnet, dass** die Einzelsegmente (18) auch in der gebogenen Endposition einen gegenseitigen Abstand aufweisen.

5. Instrument nach einem der Ansprüche 1 bis 4, **dadurch gekennzeichnet, dass** das Verbindungsmittel (16) ein Federelement ist.

6. Instrument nach einem der Ansprüche 1 bis 5, **dadurch gekennzeichnet, dass** das Verbindungsmittel (16) aus einem Metall mit Memory-Effekt, vorzugsweise aus Nitinol, besteht oder ein Metall mit Memory-Effekt, vorzugsweise Nitinol, aufweist.

7. Instrument nach einem der Ansprüche 1 bis 6, **dadurch gekennzeichnet, dass** das Verbindungsmittel (16) aus mindestens zwei seitlich beabstandeten Elementen besteht.

8. Instrument nach einem der Ansprüche 1 bis 7, **dadurch gekennzeichnet, dass** das Verbindungsmittel (16) an der im gebogenen Zustand äußeren Seite der Einzelsegmente (18) angeordnet ist.

9. Instrument nach einem der Ansprüche 1- 8, **dadurch gekennzeichnet, dass** das Zugmittel (10) auf der von dem Verbindungsmittel (16) entfernten Seite der Einzelsegmente (18) hindurchgeführt ist.

10. Instrument nach einem der Ansprüche 1 bis 9, **dadurch gekennzeichnet, dass** der maximale Verschiebungsweg des Zugmittels (10) durch ein Betätigungsmittel (22, 24) im Bedienungshandgriff (2) begrenzt ist.

11. Instrument nach Anspruch 10, **dadurch gekennzeichnet, dass** der maximale Verschiebungsweg des Zugmittels (10) variabel an dem Betätigungsmittel (22, 24) einstellbar ist.

12. Instrument nach einem der Ansprüche 1 bis 11, **dadurch gekennzeichnet, dass** eine Arretiervorrichtung (26) vorgesehen ist, die in wählbaren Zugpositionen das Zugmittel (10) arretiert.

13. Instrument nach Anspruch 1 bis 12, **dadurch gekennzeichnet, dass** das der biegsame zweite Schaftabschnitt (6) auswechselbar an dem ersten Schaftabschnitt (4) befestigt ist.

## Claims

1. An instrument (1) for laparoscopic surgery, comprising
- an operating handle (2),
- a rectilinear first shaft section (4) connected to the operating handle (2),
- a flexible second shaft section (6) arranged at the distal end of the first shaft section (4) and having an end part (8) located at the distal tip of the second shaft section (6), and
- a tensioning means, wherein
- the second shaft section (6) is moveable from a rectilinear extension position (12) to a curved position (14) in a predetermined plane of curvature with the aid of the tensioning means (10) engaging on the end part (8),
wherein, between the first shaft section (4) and the end part (8), the second shaft section (6) is formed by a plurality of individual segments (18) which are moveable relative to each other in an articulated manner, wherein each individual segment (18) has a base portion (28) arranged thereon, wherein the base portions (28) of the individual segments (18) are arranged adjacent to each other while forming a joint, and
wherein the instrument is configured such that in the second shaft section (6), the instrument has a higher lateral stability in a direction orthogonal to the plane of curvature of the second shaft section (6) that in the direction of the plane of curvature,
**characterized in that**
a flexible connecting means (16) extends between the first shaft section (4) and the end part (8), and
the flexible connecting means (16) has a restoring function with which the flexible second shaft section (6) can be restored automatically to a rectilinear or predetermined extension position.
wherein the connecting means (16) is fitted through the base portions (28) of the individual segments (18).

2. The instrument of claim 1, **characterized in that** the connecting means (16) is connected to the first shaft section (4) and the end part (8) in a rigid manner.

3. The instrument of claim 1 or 2, **characterized in that** the individual segments (18) have a mutual distance.

4. The instrument of any one of claims 1 to 3, **characterized in that** the individual segments (18) have a mutual distance also in the curved end position.

5. The instrument of any one of claims 1 to 4, **characterized in that** the connecting means (16) is a spring element.

6. The instrument of any one of claims 1 to 5, **characterized in that** the connecting means (16) is made of a metal with memory effect, preferably of nitinol, or comprises a metal with memory effect, preferably nitinol.

7. The instrument of any one of claims 1 to 6, **characterized in that** the connecting means (16) comprises at least two elements which are laterally spaced from each other.

8. The instrument of any one of claims 1 to 7, **characterized in that** the connecting means (16) is arranged on that side of the individual segments (18) which in the bent state is the outer side.

9. The instrument of any one of claims 1 to 8, **characterized in that** the tensioning means (10) is fitted through on that side of the individual segments (18) which is remote from the connecting means (16).

10. The instrument of any one of claims 1 to 9, **characterized in that** the maximal displacement path of the tensioning means (10) is delimited by an operating means (22,24) in the operating handle (2).

11. The instrument of claim 10, **characterized in that** the maximal displacement path of the tensioning means (10) is variably adjustable at the actuating means (22,24).

12. The instrument of any one of claims 1 to 11, **characterized in that** a locking means (26) is provided for locking the tensioning means (10) in selectable tensioning positions.

13. The instrument of any one of claims 1 to 12, **characterized in that** the flexible second shaft section (6) is exchangeably fastened to the first shaft section (4).

## Revendications

1. Instrument (1) de chirurgie laparoscopique, doté
- d'une poignée de manœuvre (2),
- d'une première section de tige rectiligne (4) reliée à la poignée de manœuvre (2),
- d'une deuxième section de tige flexible (6) à l'extrémité distale de la première section de tige (4), laquelle comporte une partie terminale (8) se trouvant à la pointe distale de la deuxième section de tige (6), et
- d'un moyen de traction, dans lequel
- la deuxième section de tige (6) peut passer d'une position en extension rectiligne (12) à une position incurvée (14) dans un plan de courbure prédéterminé à l'aide du moyen de traction (10) agissant sur la partie terminale (8),
dans lequel la deuxième section de tige (6) est formée de plusieurs segments individuels (18) assemblés et mobiles de manière articulée l'un par rapport à l'autre entre la première section de tige (4) et la partie terminale (8), dans lequel une partie de base (28) est disposée sur chaque segment individuel (18), dans lequel les parties de base (28) des segments individuels (18) sont contiguës pour former une articulation, et
dans lequel l'instrument est réalisé de telle sorte que l'instrument présente, dans la deuxième section de tige (6), une stabilité latérale plus élevée dans la direction orthogonale au plan de courbure de la deuxième section de tige (6) que dans la direction du plan de courbure
**caractérisé en ce qu'**un moyen de liaison flexible (16) s'étend entre la première section de tige (4) et la partie terminale (8), et
le moyen de liaison flexible (16) comporte une fonction de retour, avec laquelle la deuxième section de tige souple (6) peut revenir par elle-même à une position d'extension rectiligne ou prédéfinie,
dans lequel le moyen de liaison (16) passe à travers les parties de base (28) des segments individuels (18).

2. Instrument selon la revendication 1, **caractérisé en ce que** le moyen de liaison (16) est fixé rigidement à la première section de tige (4) et à la partie terminale (8) .

3. Instrument selon la revendication 1 ou 2, **caractérisé en ce que** les segments individuels (18) présentent un écart égal l'un par rapport à l'autre.

4. Instrument selon l'une des revendications 1 à 3, **caractérisé en ce que** les segments individuels (18) présentent un écart égal l'un par rapport à l'autre également dans la position incurvée finale.

5. Instrument selon l'une des revendications 1 à 4, **caractérisé en ce que** le moyen de liaison (16) est un élément de ressort.

6. Instrument selon l'une des revendications 1 à 5, **caractérisé en ce que** le moyen de liaison (16) est constitué d'un métal à mémoire de forme, de préférence de nitinol, ou comporte un métal à mémoire de forme, de préférence du nitinol.

7. Instrument selon l'une des revendications 1 à 6, **caractérisé en ce que** le moyen de liaison (16) est constitué d'au moins deux éléments écartés latéralement.

8. Instrument selon l'une des revendications 1 à 7, **caractérisé en ce que** le moyen de liaison (16) est disposé, dans l'état incurvé, sur la face extérieure des segments individuels (18).

9. Instrument selon l'une des revendications 1- 8, **caractérisé en ce que** le moyen de traction (10) passe sur la face des segments individuels (18) qui est éloignée du moyen de liaison (16).

10. Instrument selon l'une des revendications 1 à 9, **caractérisé en ce que** la course de déplacement maximale du moyen de traction (10) est délimitée par un moyen d'actionnement (22, 24) dans la poignée de manœuvre (2).

11. Instrument selon la revendication 10, **caractérisé en ce que** la course de déplacement maximale du moyen de traction (10) est réglable de manière variable sur le moyen d'actionnement (22, 24).

12. Instrument selon l'une des revendications 1 à 11, **caractérisé en ce qu'**un dispositif de verrouillage (26) est prévu, lequel verrouille le moyen de traction (10) dans des positions de traction sélectionnables.

13. Instrument selon l'une des revendications 1 à 12, **caractérisé en ce que** la deuxième section de tige souple (6) est fixée de manière amovible à la première section de tige (4).
